# EUROPEAN PATENT APPLICATION

(11) **EP 4 435 003 A1**
(43) Date of publication of application: **25.09.2024**
(21) Application number: 22916635.0
(22) Date of filing: 23.12.2022
(51) Int. Cl.: C07K 16/24, A61P 17/14, A61K 39/00

(54) **ANTIBODY TO CXCL 12 AND COMPOSITION COMPRISING SAME FOR TREATMENT OF HAIR LOSS**

(30) Priority: 27.12.2021 KR 20210188304
(71) Applicant: Epi Biotech Co., Ltd., Incheon 21984 (KR)
(72) Inventor: ZHENG, Mei, Incheon 21658 (KR)
(74) Representative: Prüfer & Partner mbB Patentanwälte · Rechtsanwälte
(86) International application number: PCT/KR2022/021227
(87) International publication number: WO 2023/128494

(57) **Abstract**

The present invention relates to a composition comprising a neutralizing antibody to CXCL12 for treatment of hair loss. The neutralizing antibody to CXCL12 inhibits CXCL12 to exhibit an effect of promoting hair growth, and thus can be advantageously used for preventing or treating hair loss.

## Description

### Technical Field

The present invention relates to a novel antibody to CXCL12 and a composition comprising the same for treatment of hair loss. Specifically, the present invention relates to a neutralizing antibody or humanized antibody to CXCL12, and the antibody inhibits CXCL12 to exhibit an effect of promoting hair growth.

### Background Art

Mouse hair grows, maintains, and falls out through a three-stage cycle: anagen, catagen, and telogen. Generally, hair falls out after going through a catagen phase, which is a stage in which hair follicles shrink as overall apoptosis of hair follicle cells occurs for 10 to 14 days after the anagen phase, and a telogen phase, which is a phase in preparation for the next anagen that lasts an average of 3 months. The reason why the length of hair varies depending on the area is because the duration of the anagen phase, which is a unique characteristic of the hair follicle, is different for each area.

Since human hair also has a certain hair growth cycle, it always maintains a certain number of hairs. However, as hair loss progresses, the dermal papilla present in the hair root become smaller, and as the dermal papilla become smaller, the thickness of the hair becomes thinner, and at the same time, the hair growth cycle becomes shorter. Therefore, as hair loss progresses, the hair becomes very thin and the hair growth cycle becomes shorter, causing it to grow a little and then fall out.

It is known that the causes of hair loss are not only genetic causes and the action of male hormones, but also a combination of factors such as severe physical and mental stress such as endocrine diseases, nutritional deficiencies, drug use, childbirth, fever, and surgery. Recently, not only male pattern hair loss, but also the number of women with hair loss is increasing due to increased stress due to changes in dietary life, social environment, and the like, and the age is also decreasing.

Currently, the most frequently used therapeutic agents for hair loss in Korea include finasteride (brand name: Propecia^{®}), dutasteride (brand name: Avodart^{®}), minoxidil (brand name: Minoxyl^{®} or Rogaine^{®}), and the like. Finasteride and dutasteride are 5α-reductase inhibitors that inhibit the conversion of male hormones (testosterone) into 5α-dihydrotestosterone (DHT). However, this product has side effects such as decreased libido, erectile dysfunction, and loss of ability to drive and perform. Meanwhile, in the case of minoxidil, the mechanism has not yet been completely elucidated, but it is a potassium channel opener that hyperpolarizes cell membranes. Minoxidil is believed to keep hair follicles healthy by increasing the supply of oxygen, blood, and nutrients to hair follicles through vasodilation and opening of potassium channels. However, the above products also have side effects such as decreased libido, erectile dysfunction, loss of ability to drive and perform, itchiness at the application site, erythema, skin irritation, and eye irritation, and unwanted hair growth may be observed in other body parts other than the head. In addition, hair transplant surgery has recently been attempted for patients with severe hair loss, but the high cost and side effects after the surgery have been pointed out as limitations.

Meanwhile, chemokines are white blood cell chemotactic factors that attract white blood cells to various tissues in the body, and the process is essential for the body's response to inflammation and infection. Chemokines and their receptors are central to the pathophysiology of immunoregulatory, inflammatory, and infectious diseases. In addition, chemokines and their receptors have recently been discovered to have specific roles in certain diseases, including autoimmune diseases, depending on the type of the chemokine and its receptor, and therapeutic approaches that regulate the activity of specific chemokines or their receptors have been proposed.

Accordingly, the present inventors made efforts to develop a novel therapeutic agent for hair loss. As a result, the present inventors found that the growth of hair follicles and hair was promoted when treated with an antibody to CXCL12, a type of chemokine, thereby completing the present invention.

### Prior Art Document

### Non-Patent Document

(Non-Patent Document 1) Tran PB et al., Chemokine receptors in the brain: a developing story. J. Comp. Neurol. 2003;457:1-6.
(Non-Patent Document 2) Zou YR et al., Function of the chemokine receptor CXCR4 in haematopoiesis and in cerebellar development. Nature. 1998;393:595-599.
(Non-Patent Document 3) Michel et al. Study of gene expression alteration in male androgenetic alopecia: evidence of predominant molecular signaling pathways. British journal of dermatology. 2017;177:1322-1336.

### Detailed Description of Invention

### Technical Problem

An object of the present invention is to provide an antibody to CXCL12.

Another object of the present invention is to provide a composition for preventing or treating hair loss, comprising an antibody to CXCL12.

### Solution to Problem

The present invention provides an antibody that specifically binds to CXCL12, a pharmaceutical composition for preventing or treating hair loss, comprising an antibody that specifically binds to CXCL12, and a polynucleotide encoding an antibody that specifically binds to CXCL12, and a vector comprising the same.

The antibody may be a monoclonal antibody that specifically binds to CXCL12, comprising a heavy chain variable region comprising CDRH1 that has a sequence having at least 90%, 95% or 99% sequence homology to the amino acid sequence of SEQ ID NO: 1; CDRH2 that has a sequence having at least 90%, 95% or 99% sequence homology to the amino acid sequence of SEQ ID NO: 2; and CDRH3 that has a sequence having at least 90%, 95% or 99% sequence homology to the amino acid sequence of SEQ ID NO: 3; and a light chain variable region comprising CDRL1 that has a sequence having at least 90%, 95% or 99% sequence homology to the amino acid sequence of SEQ ID NO: 4; CDRL2 that has a sequence having at least 90%, 95% or 99% sequence homology to the amino acid sequence of SEQ ID NO: 5; and CDRL3 that has a sequence having at least 90%, 95% or 99% sequence homology to the amino acid sequence of SEQ ID NO: 6, and preferably, it may be a monoclonal antibody that specifically binds to CXCL12, comprising a heavy chain variable region comprising CDRH1 having the amino acid sequence of SEQ ID NO: 1; CDRH2 having the amino acid sequence of SEQ ID NO: 2; and CDRH3 having the amino acid sequence of SEQ ID NO: 3; and a light chain variable region comprising CDRL1 having the amino acid sequence of SEQ ID NO: 4; CDRL2 having the amino acid sequence of SEQ ID NO: 5; and CDRL3 having the amino acid sequence of SEQ ID NO: 6.

The antibody may be a humanized antibody that specifically binds to CXCL12, comprising a heavy chain variable region comprising CDRH1 that has a sequence having at least 90%, 95% or 99% sequence homology to the amino acid sequence of SEQ ID NO: 1; CDRH2 that has a sequence having at least 90%, 95% or 99% sequence homology to the amino acid sequence of SEQ ID NO: 2; and CDRH3 that has a sequence having at least 90%, 95% or 99% sequence homology to the amino acid sequence of SEQ ID NO: 3; and a light chain variable region comprising CDRL1 that has a sequence having at least 90%, 95% or 99% sequence homology to the amino acid sequence of SEQ ID NO: 4; CDRL2 that has a sequence having at least 90%, 95% or 99% sequence homology to the amino acid sequence of SEQ ID NO: 5; and CDRL3 that has a sequence having at least 90%, 95% or 99% sequence homology to the amino acid sequence of SEQ ID NO: 6, and preferably, it may be a humanized antibody that specifically binds to CXCL12, comprising a heavy chain variable region comprising CDRH1 having the amino acid sequence of SEQ ID NO: 1; CDRH2 having the amino acid sequence of SEQ ID NO: 2; and CDRH3 having the amino acid sequence of SEQ ID NO: 3; and a light chain variable region comprising CDRL1 having the amino acid sequence of SEQ ID NO: 4; CDRL2 having the amino acid sequence of SEQ ID NO: 5; and CDRL3 having the amino acid sequence of SEQ ID NO: 6.

The antibody may be a humanized antibody that specifically binds to CXCL12, comprising a heavy chain variable region comprising a sequence having at least 90%, 95% or 99% sequence homology to any one of the amino acid sequences of SEQ ID NO: 7 to SEQ ID NO: 9; and a light chain variable region comprising a sequence having at least 90%, 95% or 99% sequence homology to any one of the amino acid sequences of SEQ ID NO: 10 to SEQ ID NO: 12, and preferably, it may be a humanized antibody that specifically binds to CXCL12, comprising a heavy chain variable region comprising any one of the amino acid sequences of SEQ ID NO: 7 to SEQ ID NO: 9; and a light chain variable region comprising any one of the amino acid sequences of SEQ ID NO: 10 to SEQ ID NO: 12.

The antibody may be a humanized antibody that specifically binds to CXCL12, comprising a heavy chain variable region comprising the amino acid sequence of SEQ ID NO: 7; and a light chain variable region comprising the amino acid sequence of SEQ ID NO: 10,
it may be a humanized antibody that specifically binds to CXCL12, comprising a heavy chain variable region comprising the amino acid sequence of SEQ ID NO: 7; and a light chain variable region comprising the amino acid sequence of SEQ ID NO: 11, and
it may be a humanized antibody that specifically binds to CXCL12, comprising a heavy chain variable region comprising the amino acid sequence of SEQ ID NO: 7; and a light chain variable region comprising the amino acid sequence of SEQ ID NO: 12.

The antibody may be a humanized antibody that specifically binds to CXCL12, comprising a heavy chain variable region comprising the amino acid sequence of SEQ ID NO: 8; and a light chain variable region comprising the amino acid sequence of SEQ ID NO: 10,

it may be a humanized antibody that specifically binds to CXCL12, comprising a heavy chain variable region comprising the amino acid sequence of SEQ ID NO: 8; and a light chain variable region comprising the amino acid sequence of SEQ ID NO: 11, and

it may be a humanized antibody that specifically binds to CXCL12, comprising a heavy chain variable region comprising the amino acid sequence of SEQ ID NO: 8; and a light chain variable region comprising the amino acid sequence of SEQ ID NO: 12.

The antibody may be a humanized antibody that specifically binds to CXCL12, comprising a heavy chain variable region comprising the amino acid sequence of SEQ ID NO: 9; and a light chain variable region comprising the amino acid sequence of SEQ ID NO: 10,

it may be a humanized antibody that specifically binds to CXCL12, comprising a heavy chain variable region comprising the amino acid sequence of SEQ ID NO: 9; and a light chain variable region comprising the amino acid sequence of SEQ ID NO: 11, and

it may be a humanized antibody that specifically binds to CXCL12, comprising a heavy chain variable region comprising the amino acid sequence of SEQ ID NO: 9; and a light chain variable region comprising the amino acid sequence of SEQ ID NO: 12.

The hair loss may be alopecia areata, hereditary androgenetic alopecia, telogen effluvium, traumatic alopecia, trichotillomania, pressure alopecia, anagen effluvium, alopecia pityroides, syphilitic alopecia, seborrheic alopecia, symptomatic alopecia, cicatricial alopecia, congenital alopecia, or hair loss due to drug side effects. In addition, the hair loss due to drug side effects may be hair loss due to an anticancer agent, an anticoagulant, an antidepressant, or a hormonal agent, and the anticancer agent may be an anticancer agent for the treatment of cancer selected from the group consisting of melanoma, epithelial cancer, breast cancer, cervical cancer, solid cancer, urothelial cancer, non-small cell lung cancer, pancreatic adenocarcinoma, kidney cancer, ovarian cancer, pancreatic cancer, prostate cancer, esophageal cancer, and gastrointestinal cancer.

The present invention provides a composition for use in the prevention or treatment of hair loss, comprising the antibody that specifically binds to CXCL12.

The present invention provides a use of a composition comprising the antibody that specifically binds to CXCL12, for the prevention or treatment of hair loss.

The present invention provides a method for treating hair loss by administering the antibody that specifically binds to CXCL12 to a subject with hair loss.

### Effects of Invention

The present invention relates to a novel antibody to CXCL12 and a composition comprising the same for treatment of hair loss. The antibody to CXCL12 inhibits CXCL12 to exhibit an effect of promoting hair growth, and thus can be advantageously used for preventing or treating hair loss.

### Brief Description of Drawings

Figures 1 and 2 show the results of hair follicle growth according to treatment with the neutralizing antibody to CXCL12 of the present invention in a human hair follicle culture model.
Figures 3 and 4 show the results of hair growth according to treatment with the neutralizing antibody to CXCL12 of the present invention in a mouse anagen induction model.
Figure 5 shows the results obtained by analyzing the antigen binding affinity of the humanized antibody to CXCL12 of the present invention.
Figures 6 and 7 show the results of hair follicle growth according to treatment with the humanized antibody to CXCL12 of the present invention in a mouse mustache culture model.
Figures 8 and 9 show the results of hair growth according to treatment with the humanized antibody to CXCL12 of the present invention in a mouse anagen induction model.
Figure 10 shows the results of hair growth according to treatment with the humanized antibody to CXCL12 of the present invention in an alopecia areata animal model.

### Best Mode for Carrying out the Invention

Hereinafter, with reference to the accompanying drawings, embodiments and examples of the present invention will be described in detail so that those of ordinary skill in the art to which the present invention belongs can easily practice the present invention. However, the present invention may be implemented in various forms and is not limited to the embodiments and examples described herein.

Throughout the present specification, when a certain part "includes" a certain component, it means that other components may be further included, rather than excluding other components, unless otherwise stated.

The present invention relates to a neutralizing antibody or humanized antibody to CXCL12, and a composition comprising an antibody to CXCL12 for treatment of hair loss.

The neutralizing antibody to CXCL12 may be a monoclonal antibody that specifically binds to CXCL12, comprising a heavy chain variable region comprising CDRH1 having the amino acid sequence of SEQ ID NO: 1; CDRH2 having the amino acid sequence of SEQ ID NO: 2; and CDRH3 having the amino acid sequence of SEQ ID NO: 3; and a light chain variable region comprising CDRL1 having the amino acid sequence of SEQ ID NO: 4; CDRL2 having the amino acid sequence of SEQ ID NO: 5; and CDRL3 having the amino acid sequence of SEQ ID NO: 6, and it may be a humanized antibody that specifically binds to CXCL12, comprising a heavy chain variable region comprising CDRH1 having the amino acid sequence of SEQ ID NO: 1; CDRH2 having the amino acid sequence of SEQ ID NO: 2; and CDRH3 having the amino acid sequence of SEQ ID NO: 3; and a light chain variable region comprising CDRL1 having the amino acid sequence of SEQ ID NO: 4; CDRL2 having the amino acid sequence of SEQ ID NO: 5; and CDRL3 having the amino acid sequence of SEQ ID NO: 6, and it may be a humanized antibody that specifically binds to CXCL12, comprising a heavy chain variable region comprising any one of the amino acid sequences of SEQ ID NO: 7 to SEQ ID NO: 9; and a light chain variable region comprising any one of the amino acid sequences of SEQ ID NO: 10 to SEQ ID NO: 12.

As used herein, the term "CXCL12 (C-X-C motif chemokine 12)" is stromal cell-derived factor 1 (SDF1), a type of chemokine, and is known to interact with CXCR4 as a ligand for the C-X-C chemokine receptor CXCR4. It has also been reported as a ligand for CXCR7 (RDCI). CXCL12 can be widely expressed in various tissue types, including heart, liver, spleen, kidney, brain, skeletal muscle, endothelial cells, epithelial tissue, and stem cells.

As used herein, the term "chemokine" refers to a basic heparin-binding low-molecular-weight protein that has white blood cell migration and activation effects. There are four cysteine residues in a chemokine molecule, and according to the form of the existence of the first two intramolecular cysteine residues, it is classified into four subfamilies: CXC (CXCL), CC (CCL), CX3C (CX3CL), and C (XCL), and currently more than 40 species have been identified.

As used herein, the term "neutralizing antibody" refers to an antibody that binds specifically to the antigen and loses or reduces its activity when the antigen, whether in particle or molecular form, exhibits biological activity such as infectiousness, toxicity, or enzymatic activity toward living organisms.

As used herein, the term "humanized antibody" is an antibody that possesses an amino acid sequence corresponding to that of an antibody produced by humans and/or has been made using techniques for making human antibodies as disclosed herein.

The "antibody" is a natural immunoglobulin or an immunoglobulin produced partially or entirely synthetically. The term also includes any polypeptide or protein comprising the antigen binding domain of an antibody. An antibody fragment comprising an antigen binding domain is a molecule such as Fab, scFv, Fv, dAb, Fd, and diabody. The term "antibody" is used in a broad sense and specifically includes an intact monoclonal antibody, a polyclonal antibody, and a multispecific antibody (for example, a bispecific antibody) formed from at least two intact antibodies. The antibody also includes an antibody fragment, so long as it exhibit the desired biological activity. The antibody consists of two heavy chains and two light chains, and has a variable region in which the amino acid sequence changes depending on the type of target antigen and a constant region in which the sequence does not change.

The variable regions of the light and heavy chains include three highly variable regions referred to as "complementarity determining region (CDR)." CDRs bind to the epitope of an antigen, and the CDRs of each chain typically have three regions (CDR1, CDR2, and CDR3).

As used herein, the term "specifically binding" is commonly known to those of ordinary skill in the art, and may specifically mean that an antigen and an antibody can specifically interact to form an antigen-antibody complex and also produce an immunological reaction.

As used herein, the term "hair loss" refers to the absence of hair in areas where hair should normally exist, regardless of the cause, and includes alopecia areata, hereditary androgenetic alopecia, telogen effluvium, traumatic alopecia, trichotillomania, pressure alopecia, anagen effluvium, alopecia pityroides, syphilitic alopecia, seborrheic alopecia, symptomatic alopecia, cicatricial alopecia, congenital alopecia, or hair loss due to drug side effects, but is not limited thereto.

As used herein, the term "prevention" refers to any action of inhibiting or delaying the onset of a disease by administration of a composition, and "treatment" refers to any action in which symptoms of a subject suspected of and suffering from a disease are improved or beneficially changed by administration of a composition.

The pharmaceutical composition of the present invention can be formulated and used in any form suitable for the preparation, including, oral dosage forms such as powders, granules, tablets, soft or hard capsules, suspensions, emulsions, syrups, and aerosols, external skin preparations such as ointments and creams, suppositories, injections, fillers, and sterilized injection solution according to conventional methods.

Hereinafter, the present invention will be described in more detail through the examples, but the following examples are for illustrative purposes only and are not intended to limit the scope of the present invention.

### [Example 1]

### Construction of neutralizing antibody to CXCL12

### 1-1. Construction of immunized mouse

50 µg (per mouse) of recombinant human CXCL12 protein (cat# 13511-HNCE) purchased from Sinobiologics was mixed with an equal volume of complete Freund's adjuvant (Sigma, USA) to prepare an emulsion. The emulsion prepared as above was injected into the abdominal cavity of six 7-week-old female BALB/C mice. 50 µg of antigen was injected into each mouse in a total volume of 500 µl. After 1 and 2 weeks, an emulsion mixed with incomplete Freund's adjuvant (Sigma, USA) and the antigen was additionally injected into the abdominal cavity of the mice.

### 1-2. Confirmation of antibody production

Blood was collected from the eyes of mice immunized using the above method, placed in a 1.5 ml microcentrifuge tube, and then centrifuged at 13,000 rpm for 10 minutes. Serum was separated and stored at -20 °C until an experiment to confirm antibody production was performed. An enzyme immunoassay using the antigen protein was performed to confirm antibody production, and then an emulsion mixed with incomplete Freund's adjuvant (Sigma, USA) and the antigen was injected into the abdominal cavity of the mice once again 3 days before cell fusion.

### 1-3. Preparation of hybridoma

After confirming antibody production, the mouse was sacrificed. Splenocytes were isolated and fused with myeloma cells P3X63Ag 8.653 (ATCC CRL-1580) to prepare a hybridoma. Specifically, the mouse P3X63Ag 8.653 cells were cultured in a culture plate using RPMI1640 medium supplemented with 10% fetal bovine serum. In order to perform cell fusion, P3X63Ag 8.653 cells were washed twice with serum-free RPMI1640 medium (Hyclone, USA) and adjusted to a cell concentration of 1 × 10⁷. The mouse was sacrificed by cervical dislocation, the spleen was collected, then placed in a mesh container (Sigma, USA), and the cells were isolated. A suspension of splenocytes was prepared, and then the suspension was washed using centrifugation. The splenocyte solution was exposed to Tris-NH4Cl (TRIS 20.6 g/L, NH4Cl 8.3 g/L) to lyse red blood cells. Completely isolated antibody-producing cells were centrifuged at 400 xg for 5 minutes. Thereafter, the cells were washed twice in serum-free medium and resuspended in 10 ml of medium. Lymphocytes were counted using a hemocytometer, and 1 × 10⁸ lymphocytes were mixed with 1 × 10 P3X63Ag 8.653 cells (10:1) in serum-free medium. After centrifugation at 400 xg for 5 minutes, 1 ml solution was added dropwise using 50% (M/V) polyethylene glycol 1500 (Sigma, USA) warmed at 37 °C and mixed for 1 minute. The fusion mixture solution prepared as above was diluted with serum-free RPMI1640 and centrifuged at 400 xg for 3 minutes. The cells were suspended in 35 ml of RPMI1640 selection medium supplemented with 20% fetal bovine serum and HAT (100 µM hypoxanthine, 0.4 µM aminopterin, 16 µM thymidine). 100 µl of the suspension was loaded onto a 96-well plate coated with feeder cells (macrophages isolated from the abdominal cavity using RPMI1640) 1 day ago, and cultured at 37 °C 5% CO₂. After 5 days, the HAT medium was replaced every 2 to 3 days, and the cells were cultured for 14 days. After 14 days, secondary culture was performed by replacing the medium with RPMI1640 medium supplemented with 20% fetal bovine serum and HT (medium with 0.4 µM aminopterin removed from HAT).

### 1-4. Selection and isolation of antibody-producing fused cells

The supernatant of the fused cell culture solution prepared as above was collected, and enzyme immunoassay was performed to determine whether antibodies specific for the prepared antigen were produced. A culture solution of the fused cells that showed an appropriate concentration of more than 4 times that of the negative control group was selected, transferred to a 24-well plate, and cultured. Additionally, the culture solution was diluted (limiting dilution) to include 1 cell per well in a 96-well plate and cultured, and then the culture solution was recovered, and the 96-well plate was coated with 0.1 µg of CXCL12 protein, used as an antigen, per well. Thereafter, enzyme immunoassay was performed to finally select the fused cells producing monoclonal antibodies.

### 1-5. Analysis of monoclonal antibody

The neutralizing antibody to CXCL12 obtained through the above process was designated as 2B12. As a result of sequence analysis, as shown in Table 1 below, it was confirmed that the above neutralizing antibody comprises a heavy chain variable region comprising CDRH1 having the sequence of SEQ ID NO: 1; CDRH2 having the sequence of SEQ ID NO: 2; and CDRH3 having the sequence of SEQ ID NO: 3; and a light chain variable region comprising CDRL1 having the sequence of SEQ ID NO: 4; CDRL2 having the sequence of SEQ ID NO: 5; and CDRL3 having the sequence of SEQ ID NO: 6.

**[Table 1]**

| Antibody | | Amino acid sequence | SEQ ID NO |
|---|---|---|---|
| 2B12 | Heavy chain CDR1 (CDRH1) | SYYIH | 1 |
| | Heavy chain CDR2 (CDRH2) | WIYPGNVNTKYNEKFKG | 2 |
| | Heavy chain CDR3 (CDRH3) | CWGYYYGS SYAMDY | 3 |
| | Light chain CDR1 (CDRL1) | RASQSIGTSIH | 4 |
| | Light chain CDR2 (CDRL2) | YASESIS | 5 |
| | Light chain CDR3 (CDRL3) | QQSNSWPTYT | 6 |

### [Example 2]

### Confirmation of hair follicle growth promotion effect according to treatment with neutralizing antibody to CXCL12 in human hair follicle culture model

In order to confirm the hair follicle growth promotion effect of the neutralizing antibody to CXCL12 constructed through Example 1 above, the procedure was performed as follows.

Hair follicles from a man in his 30s were trimmed, and organ culture was performed. Hair follicles were cultured at one hair follicle per well in a 48-well plate, and divided into a total of 6 groups: negative control group, positive control group (commercially available antibody 50, 500 ng/mL), and the group treated with 2B12 (0.05, 0.5, 5 ng/mL), and human hair follicles were treated and then cultured in an incubator for 4 days.

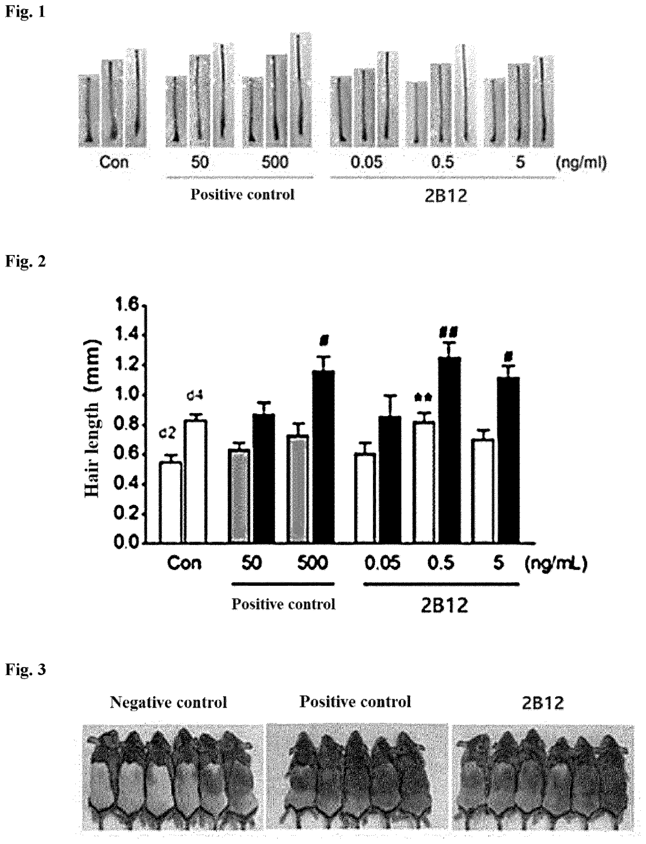

As shown in Figures 1 and 2, as a result of the culture experiment of human hair follicles, it was confirmed that the growth of hair follicles was promoted when treated with the neutralizing antibody 2B 12 to CXCL12 of the present invention, and it was confirmed that this was at a level similar to or better than that of the positive control group.

Therefore, it can be seen that the neutralizing antibody to CXCL12 of the present invention has an excellent hair growth effect by promoting the growth of hair follicles by inhibiting CXCL12.

### [Example 3]

### Confirmation of hair growth promotion effect according to treatment with neutralizing antibody to CXCL12 in mouse anagen induction model

In order to confirm the hair growth promotion effect of the neutralizing antibody to CXCL12 constructed through Example 1 above, the procedure was performed as follows.

Seventeen 7-week-old male C₃H/HeN mice were shaved short using a hair removal machine, and the remaining hair was completely removed using a depilatory, and then the mice were stabilized for one day before being used in the experiment. The experiment was performed with 5 to 6 mice in each group, and the mice were divided into a total of 4 groups: negative control group (IgG1, 5 µg/head), positive control group (commercially available antibody 5 µg/head) and the group administered with 2B 12 antibody (5 µg/head). Each group was subcutaneously injected into the back of the mouse a total of 4 times at intervals of 4 days (total dosage 20 µg/head), and the degree of hair growth on the back of the mouse was monitored for 17 days after injection of each antibody. After 17 days, the back of the mouse where hair growth had occurred was shaved with a razor, then the hair was obtained, and its weight was measured.

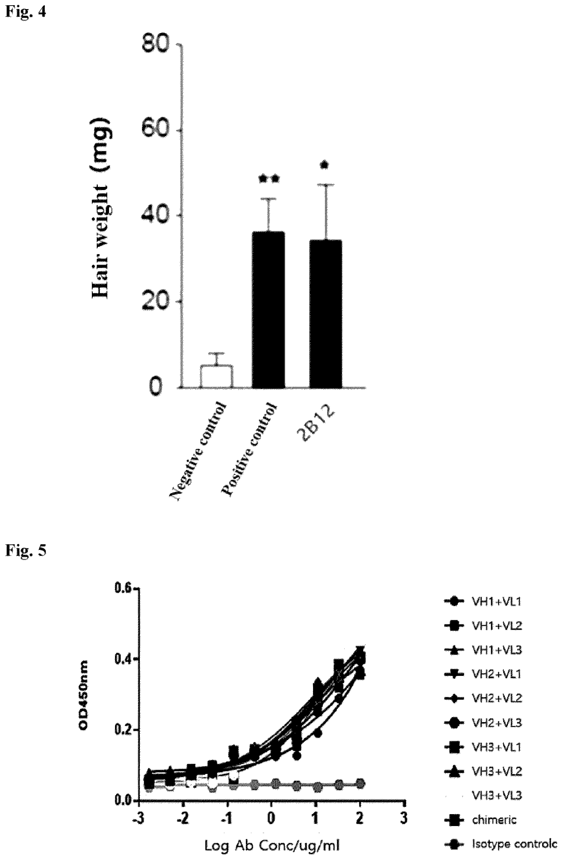

As shown in Figures 3 and 4, as a result of evaluating the hair growth promotion effect in the mice, it was confirmed that the hair growth was promoted when the neutralizing antibody 2B 12 to CXCL12 of the present invention was administered.

Therefore, it can be seen that the neutralizing antibody to CXCL12 of the present invention has an excellent hair growth effect by promoting hair growth by inhibiting CXCL12.

### [Example 4]

### Construction of humanized antibody based on neutralizing antibody to CXCL12

### 4-1. Construction of humanized antibody

In order to construct a humanized antibody by changing the neutralizing antibody to CXCL12 of Example 1 above into a structure corresponding to a human, it was constructed by a CDR grafting method in which the CDRs of a mouse antibody are grafted onto a human antibody. The results of analyzing the sequence of the constructed humanized antibody are shown in Table 2 below.

**[Table 2]**

| Antibody | | Amino acid sequence | SEQ ID NO |
|---|---|---|---|
| Heavy chain | VH1 | QVQLVQSGAEVKKPGASVKVSCKASGYTFTSYYIHWVR QAPGQRLEWIGWIYPGNVNTKYNEKFKGRVTITADTSAS TAYMELSSLRSEDTAVYYCARCWGYYYGSSYAMDYWG QGTLVTVSS | 7 |
| | VH2 | QVQLVQSGAEVKKPGASVKVSCKASGYTFTSYYIHWVR QAPGQRLEWIGWIYPGNVNTKYNEKFKGRATITADTSAS TAYMELSSLRSEDTAVYFCARCWGYYYGSSYAMDYWG QGTLVTVSS | 8 |
| | VH3 | QVQLVQSGAEVKKPGASVKVSCKASGYTFTSYYIHWVR QAPGQGLEWIGWIYPGNVNTKYNEKFKGRATITADTSAS TAYMELSSLRSEDTAVYFCARCWGYYYGSSYAMDYWG QGTLVTVSS | 9 |
| Light chain | VI,1 | EIVLTQSPDFQSVTPKEKVTITCRASQSIGTSIHWYQQKPD QSPKLLIKYASESISGVPSRFSGSGSGTDFTLTINSLEAEDA ATYYCQQSNSWPTYTFGGGTKLEIK | 10 |
| | VL2 | EIVLTQSPDFQSVTPKEKVTITCRASQSIGTSIHWYQQKPD QSPRLLIKYASESISGVPSRFSGSGSGTDFTLTINSLEAEDA ADYYCQQSNSWPTYTFGGGTKLEIK | 11 |
| | VL3 | EIVLTQSPDFQSVTPKEKVTFTCRASQSIGTSIHWYQQKP DQSPRLLIKYASESISGIPSRFSGSGSGTDFTLTINSLEAED AADYYCQQSNSWPTYTFGGGTKLEIK | 12 |

### 4-2. Construction and production of humanized IgG

In order to construct a humanized IgG antibody, the DNA sequences encoding the heavy and light chains of chimeric and humanized antibodies were synthesized and inserted into the pcDNA3.4 vector to construct an expression plasmid for full-length hIgG. The heavy and light chain plasmids were transfected according to GenScriptProbio's standard operating procedures (SOP), and antibody expression was performed in Expi293F cell culture. As shown in Table 3 below, a total of 9 combinations of humanized antibodies for heavy and light chain variable regions were expressed.

**[Table 3]**

| No. | Antibody |
|---|---|
| 1 | VH1 + VL1 |
| 2 | VH1 + VL2 |
| 3 | VH1 + VL3 |
| 4 | VH2 + VL1 |
| 5 | VH2 + VL2 |
| 6 | VH2 + VL3 |
| 7 | VH3 + VL1 |
| 8 | VH3 + VL2 |
| 9 | VH3 + VL3 |

### 4-3. Confirmation of antigen binding affinity of purified humanized IgG (ELISA)

In order to confirm the antigen binding affinity of the nine combinations of humanized antibodies above, the procedure was performed as follows.

The initial concentration of all antibodies was 100 µg/ml, serial diluted by 1/3, cultured O/N at 4°C to make 100 µl per well in a well plate, and cultured at 37 °C for 1 hour with 3% Nonfat Milk the next day. Thereafter, the plate was washed with washing buffer and then cultured with 100 µl of antigen (SDF-1α) at a concentration of 2 µg/ml antigen for 2 hours at room temperature. After 2 hours, the well plate was washed with washing buffer and then treated by addition of secondary antibody (0.1 µg/ml His-[HRP]) for 45 minutes. Thereafter, 100 µl of TMB-substrate was added to the washed well plate and reacted for 10 minutes, and then 50 µl of 1M HCL was added to stop the reaction. The absorbance was measured at 450 nm using a spectrophotometer, and binding affinity was analyzed.

As shown in Figure 5, as a result of analyzing the antigen binding affinity, it was found that the nine combinations of humanized antibodies had excellent antigen binding affinity, and in particular, the four humanized antibodies had excellent antigen binding affinity compared to a chimeric antibody.

### [Example 5]

### Confirmation of hair follicle growth promotion effect according to treatment with humanized antibody to CXCL12 in mouse mustache model

In order to confirm the hair follicle growth promotion effect of the humanized antibody to CXCL12 constructed through Example 4 above, the procedure was performed as follows.

The bristles of C57BL/6 female mice were trimmed, and organ culture was performed. Hair follicles were cultured at one hair follicle per well in a 48-well plate, and divided into a total of 21 groups: negative control group, the group treated with chimeric antibody (50, 500 ng/mL), and the group treated with IgG humanized antibody (50, 500 ng/mL), and the bristles were each treated, and then cultured in an incubator at 37 °C for 2 days.

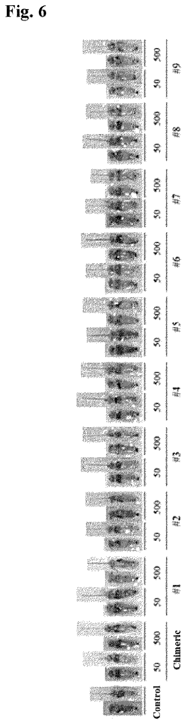

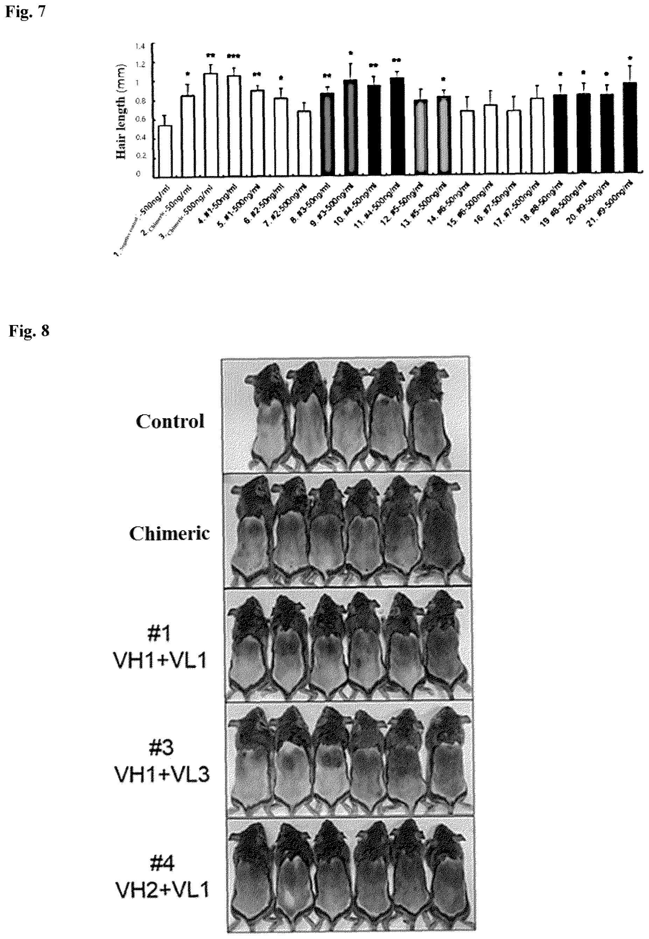

As shown in Figures 6 and 7, as a result of the culture experiment of mouse hair follicles, it was confirmed that the growth of hair follicles was promoted in all cases treated with the humanized antibody to CXCL12 of the present invention.

Therefore, it was confirmed that the humanized antibody to CXCL12 of the present invention has an excellent hair growth effect by promoting the growth of hair follicles by inhibiting CXCL12.

### [Example 6]

### Confirmation of hair growth promotion effect according to treatment with humanized antibody to CXCL12 in mouse anagen induction model

In order to confirm the hair growth promotion effect of the humanized antibody to CXCL12 constructed through Example 4 above, the procedure was performed as follows.

Twenty-three 7-week-old male C₃H/HeN mice were shaved short using a hair removal machine, and the remaining hair was completely removed using a depilatory, and then the mice were stabilized for one day. The experiment was performed with 5 to 6 mice in each group, and the groups were as follows.
Group 1: control group (IgG1, 50 µg/head), 5 mice
Group 2: group administered with chimeric antibody (50 µg/head), 6 mice
Group 3: group administered with (#1) VH1-VL1 antibody (50 µg/head), 6 mice
Group 4: group administered with (#3) VH1-VL3 antibody (50 µg/head), 6 mice
Group 5: group administered with (#4) VH2-VL1 antibody (50 µg/head), 6 mice

Each group was subcutaneously injected into the back of the mouse once (dosage 50 µg/head), and the degree of hair growth on the back of the mouse was monitored for 17 days after injection of each antibody. After 17 days, the back of the mouse where hair growth had occurred was shaved with a razor, then the hair was obtained, and its weight was measured.

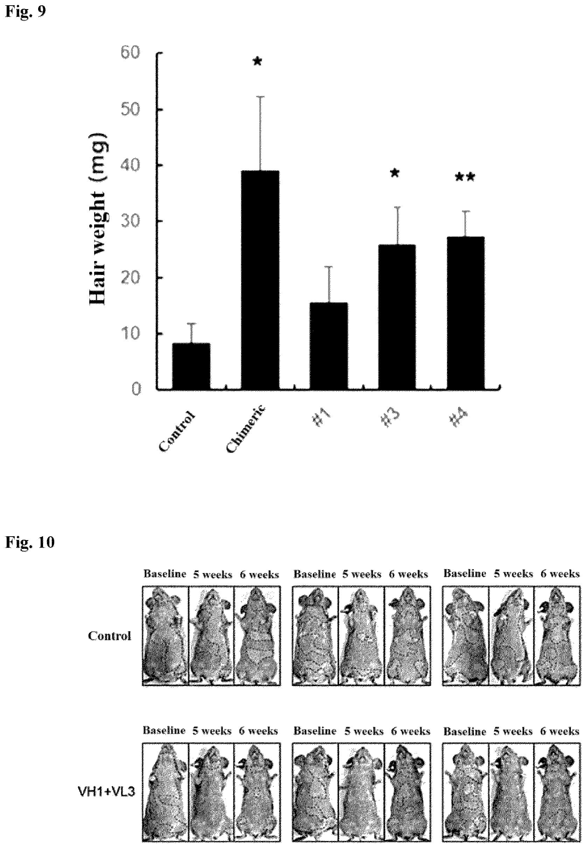

As shown in Figures 8 and 9, as a result of evaluating the hair growth promotion effect in the mice, it was confirmed that the hair growth was promoted when treated with the humanized antibody to CXCL12 of the present invention.

Therefore, it was confirmed that the humanized antibody to CXCL12 of the present invention has an excellent hair growth effect by promoting hair growth by inhibiting CXCL12.

### [Example 7]

### Confirmation of hair growth promotion effect according to treatment with humanized antibody to CXCL12 in alopecia areata animal model

In order to confirm the hair growth promotion effect of the humanized antibody to CXCL12 constructed through Example 4 above, the procedure was performed as follows.

Eddy His Chun Wang et al. conducted an experiment by obtaining an alopecia areata animal model by transplanting skin-draining lymph node cells (LNC) derived from alopecia areata animals in a 10-week-old C3H/HeJ animal model. The experiment was conducted with three animals in each group, and the groups were as follows.
Group 1: control group (IgG1, 100 µg/head), 3 mice
Group 2: group administered with VH1-VL3 antibody (100 µg/head), 3 mice

On the 1st and 10th days after the start of the experiment, a total of two subcutaneous injections were made into the abdominal skin of the mice for each group (dosage 50 µg/head/time). The skin and hair growth status of the mice was observed by taking pictures of the hair loss area for 6 weeks.

As shown in Figure 10, as a result of evaluating the hair growth promotion effect in an alopecia areata animal model, it was confirmed that the hair growth was promoted when treated with the humanized antibody to CXCL12 of the present invention.

Therefore, it was confirmed that the humanized antibody to CXCL12 of the present invention has an excellent hair growth effect by promoting hair growth by inhibiting CXCL12, and in particular, it was confirmed that it has an effect of treating alopecia areata.

## Claims

1. A monoclonal antibody that specifically binds to CXCL12, comprising
a heavy chain variable region comprising CDRH1 that has a sequence having at least 90% sequence homology to the amino acid sequence of SEQ ID NO: 1; CDRH2 that has a sequence having at least 90% sequence homology to the amino acid sequence of SEQ ID NO: 2; and CDRH3 that has a sequence having at least 90% sequence homology to the amino acid sequence of SEQ ID NO: 3; and
a light chain variable region comprising CDRL1 that has a sequence having at least 90% sequence homology to the amino acid sequence of SEQ ID NO: 4; CDRL2 that has a sequence having at least 90% sequence homology to the amino acid sequence of SEQ ID NO: 5; and CDRL3 that has a sequence having at least 90% sequence homology to the amino acid sequence of SEQ ID NO: 6.

2. The monoclonal antibody that specifically binds to CXCL12 according to claim 1, wherein the monoclonal antibody that specifically binds to CXCL12 comprises
a heavy chain variable region comprising CDRH1 that has a sequence having at least 95% sequence homology to the amino acid sequence of SEQ ID NO: 1; CDRH2 that has a sequence having at least 95% sequence homology to the amino acid sequence of SEQ ID NO: 2; and CDRH3 that has a sequence having at least 95% sequence homology to the amino acid sequence of SEQ ID NO: 3; and
a light chain variable region comprising CDRL1 that has a sequence having at least 95% sequence homology to the amino acid sequence of SEQ ID NO: 4; CDRL2 that has a sequence having at least 95% sequence homology to the amino acid sequence of SEQ ID NO: 5; and CDRL3 that has a sequence having at least 95% sequence homology to the amino acid sequence of SEQ ID NO: 6.

3. The monoclonal antibody that specifically binds to CXCL12 according to claim 1, wherein the monoclonal antibody that specifically binds to CXCL12 comprises
a heavy chain variable region comprising CDRH1 that has a sequence having at least 99% sequence homology to the amino acid sequence of SEQ ID NO: 1; CDRH2 that has a sequence having at least 99% sequence homology to the amino acid sequence of SEQ ID NO: 2; and CDRH3 that has a sequence having at least 99% sequence homology to the amino acid sequence of SEQ ID NO: 3; and
a light chain variable region comprising CDRL1 that has a sequence having at least 99% sequence homology to the amino acid sequence of SEQ ID NO: 4; CDRL2 that has a sequence having at least 99% sequence homology to the amino acid sequence of SEQ ID NO: 5; and CDRL3 that has a sequence having at least 99% sequence homology to the amino acid sequence of SEQ ID NO: 6.

4. The monoclonal antibody that specifically binds to CXCL12 according to claim 1, wherein the monoclonal antibody that specifically binds to CXCL12 comprises
a heavy chain variable region comprising CDRH1 having the amino acid sequence of SEQ ID NO: 1; CDRH2 having the amino acid sequence of SEQ ID NO: 2; and CDRH3 having the amino acid sequence of SEQ ID NO: 3; and
a light chain variable region comprising CDRL1 having the amino acid sequence of SEQ ID NO: 4; CDRL2 having the amino acid sequence of SEQ ID NO: 5; and CDRL3 having the amino acid sequence of SEQ ID NO: 6.

5. A humanized antibody that specifically binds to CXCL12, comprising
a heavy chain variable region comprising CDRH1 that has a sequence having at least 90% sequence homology to the amino acid sequence of SEQ ID NO: 1; CDRH2 that has a sequence having at least 90% sequence homology to the amino acid sequence of SEQ ID NO: 2; and CDRH3 that has a sequence having at least 90% sequence homology to the amino acid sequence of SEQ ID NO: 3; and
a light chain variable region comprising CDRL1 that has a sequence having at least 90% sequence homology to the amino acid sequence of SEQ ID NO: 4; CDRL2 that has a sequence having at least 90% sequence homology to the amino acid sequence of SEQ ID NO: 5; and CDRL3 that has a sequence having at least 90% sequence homology to the amino acid sequence of SEQ ID NO: 6.

6. The humanized antibody that specifically binds to CXCL12 according to claim 5, wherein the humanized antibody that specifically binds to CXCL12 comprises
a heavy chain variable region comprising CDRH1 that has a sequence having at least 95% sequence homology to the amino acid sequence of SEQ ID NO: 1; CDRH2 that has a sequence having at least 95% sequence homology to the amino acid sequence of SEQ ID NO: 2; and CDRH3 that has a sequence having at least 95% sequence homology to the amino acid sequence of SEQ ID NO: 3; and
a light chain variable region comprising CDRL1 that has a sequence having at least 95% sequence homology to the amino acid sequence of SEQ ID NO: 4; CDRL2 that has a sequence having at least 95% sequence homology to the amino acid sequence of SEQ ID NO: 5; and CDRL3 that has a sequence having at least 95% sequence homology to the amino acid sequence of SEQ ID NO: 6.

7. The humanized antibody that specifically binds to CXCL12 according to claim 5, wherein the humanized antibody that specifically binds to CXCL12 comprises
a heavy chain variable region comprising CDRH1 that has a sequence having at least 99% sequence homology to the amino acid sequence of SEQ ID NO: 1; CDRH2 that has a sequence having at least 99% sequence homology to the amino acid sequence of SEQ ID NO: 2; and CDRH3 that has a sequence having at least 99% sequence homology to the amino acid sequence of SEQ ID NO: 3; and
a light chain variable region comprising CDRL1 that has a sequence having at least 99% sequence homology to the amino acid sequence of SEQ ID NO: 4; CDRL2 that has a sequence having at least 99% sequence homology to the amino acid sequence of SEQ ID NO: 5; and CDRL3 that has a sequence having at least 99% sequence homology to the amino acid sequence of SEQ ID NO: 6.

8. The humanized antibody that specifically binds to CXCL12 according to claim 5, wherein the humanized antibody that specifically binds to CXCL12 comprises
a heavy chain variable region comprising CDRH1 having the amino acid sequence of SEQ ID NO: 1; CDRH2 having the amino acid sequence of SEQ ID NO: 2; and CDRH3 having the amino acid sequence of SEQ ID NO: 3; and
a light chain variable region comprising CDRL1 having the amino acid sequence of SEQ ID NO: 4; CDRL2 having the amino acid sequence of SEQ ID NO: 5; and CDRL3 having the amino acid sequence of SEQ ID NO: 6.

9. A humanized antibody that specifically binds to CXCL12, comprising
a heavy chain variable region comprising a sequence having at least 90% sequence homology to any one of the amino acid sequences of SEQ ID NO: 7 to SEQ ID NO: 9; and
a light chain variable region comprising a sequence having at least 90% sequence homology to any one of the amino acid sequences of SEQ ID NO: 10 to SEQ ID NO: 12.

10. The humanized antibody that specifically binds to CXCL12 according to claim 9, wherein the humanized antibody that specifically binds to CXCL12 comprises
a heavy chain variable region comprising a sequence having at least 95% sequence homology to any one of the amino acid sequences of SEQ ID NO: 7 to SEQ ID NO: 9; and
a light chain variable region comprising a sequence having at least 95% sequence homology to any one of the amino acid sequences of SEQ ID NO: 10 to SEQ ID NO: 12.

11. The humanized antibody that specifically binds to CXCL12 according to claim 9, wherein the humanized antibody that specifically binds to CXCL12 comprises
a heavy chain variable region comprising a sequence having at least 99% sequence homology to any one of the amino acid sequences of SEQ ID NO: 7 to SEQ ID NO: 9; and
a light chain variable region comprising a sequence having at least 99% sequence homology to any one of the amino acid sequences of SEQ ID NO: 10 to SEQ ID NO: 12.

12. The humanized antibody that specifically binds to CXCL12 according to claim 9, wherein the humanized antibody that specifically binds to CXCL12 comprises
a heavy chain variable region comprising any one of the amino acid sequences of SEQ ID NO: 7 to SEQ ID NO: 9; and
a light chain variable region comprising any one of the amino acid sequences of SEQ ID NO: 10 to SEQ ID NO: 12.

13. The humanized antibody that specifically binds to CXCL12 according to claim 9, wherein the humanized antibody that specifically binds to CXCL12 comprises a heavy chain variable region comprising the amino acid sequence of SEQ ID NO: 7; and a light chain variable region comprising the amino acid sequence of SEQ ID NO: 10.

14. The humanized antibody that specifically binds to CXCL12 according to claim 9, wherein the humanized antibody that specifically binds to CXCL12 comprises a heavy chain variable region comprising the amino acid sequence of SEQ ID NO: 7; and a light chain variable region comprising the amino acid sequence of SEQ ID NO: 11.

15. The humanized antibody that specifically binds to CXCL12 according to claim 9, wherein the humanized antibody that specifically binds to CXCL12 comprises a heavy chain variable region comprising the amino acid sequence of SEQ ID NO: 7; and a light chain variable region comprising the amino acid sequence of SEQ ID NO: 12.

16. The humanized antibody that specifically binds to CXCL12 according to claim 9, wherein the humanized antibody that specifically binds to CXCL12 comprises a heavy chain variable region comprising the amino acid sequence of SEQ ID NO: 8; and a light chain variable region comprising the amino acid sequence of SEQ ID NO: 10.

17. The humanized antibody that specifically binds to CXCL12 according to claim 9, wherein the humanized antibody that specifically binds to CXCL12 comprises a heavy chain variable region comprising the amino acid sequence of SEQ ID NO: 8; and a light chain variable region comprising the amino acid sequence of SEQ ID NO: 11.

18. The humanized antibody that specifically binds to CXCL12 according to claim 9, wherein the humanized antibody that specifically binds to CXCL12 comprises a heavy chain variable region comprising the amino acid sequence of SEQ ID NO: 8; and a light chain variable region comprising the amino acid sequence of SEQ ID NO: 12.

19. The humanized antibody that specifically binds to CXCL12 according to claim 9, wherein the humanized antibody that specifically binds to CXCL12 comprises a heavy chain variable region comprising the amino acid sequence of SEQ ID NO: 9; and a light chain variable region comprising the amino acid sequence of SEQ ID NO: 10.

20. The humanized antibody that specifically binds to CXCL12 according to claim 9, wherein the humanized antibody that specifically binds to CXCL12 comprises a heavy chain variable region comprising the amino acid sequence of SEQ ID NO: 9; and a light chain variable region comprising the amino acid sequence of SEQ ID NO: 11.

21. The humanized antibody that specifically binds to CXCL12 according to claim 9, wherein the humanized antibody that specifically binds to CXCL12 comprises a heavy chain variable region comprising the amino acid sequence of SEQ ID NO: 9; and a light chain variable region comprising the amino acid sequence of SEQ ID NO: 12.

22. A pharmaceutical composition for preventing or treating hair loss, comprising the antibody according to any one of claims 1 to 21.

23. The pharmaceutical composition for preventing or treating hair loss according to claim 22, wherein the hair loss is selected from the group consisting of alopecia areata, hereditary androgenetic alopecia, telogen effluvium, traumatic alopecia, trichotillomania, pressure alopecia, anagen effluvium, alopecia pityroides, syphilitic alopecia, seborrheic alopecia, symptomatic alopecia, cicatricial alopecia, congenital alopecia, and hair loss due to drug side effects.

24. The pharmaceutical composition for preventing or treating hair loss according to claim 23, wherein the hair loss due to drug side effects is hair loss due to a drug selected from the group consisting of an anticancer agent, an anticoagulant, an antidepressant, and a hormonal agent.

25. The pharmaceutical composition for preventing or treating hair loss according to claim 24, wherein the anticancer agent is an anticancer agent for the treatment of cancer selected from the group consisting of melanoma, epithelial cancer, breast cancer, cervical cancer, solid cancer, urothelial cancer, non-small cell lung cancer, pancreatic adenocarcinoma, kidney cancer, ovarian cancer, pancreatic cancer, prostate cancer, esophageal cancer, and gastrointestinal cancer.

26. A polynucleotide encoding the antibody according to any one of claims 1 to 21.

27. A vector comprising the polynucleotide according to claim 26.
